# EUROPEAN PATENT APPLICATION

(11) **EP 2 944 325 A1**
(43) Date of publication of application: **18.11.2015**
(21) Application number: 14305726.3
(22) Date of filing: 16.05.2014
(51) Int. Cl.: A61K 47/48, A61K 49/00, C07D 271/04

(54) **Sydnone derivatives for conjugation of compounds of interest**

(71) Applicant: Université de Strasbourg, 67000 Strasbourg (FR); Centre National de la Recherche Scientifique, 75016 Paris (FR); COMMISSARIAT A L'ENERGIE ATOMIQUE ET AUX ENERGIES ALTERNATIVES, 75015 Paris (FR)
(72) Inventor: Taran, Frédéric, 91190 GIF SUR YVETTE (FR); Wagner, Alain, 67000 STRASBOURG (FR); Kolodych, Sergii, 91400 ORSAY (FR); Koniev, Oleksandr, 67000 STRASBOURG (FR)
(74) Representative: Tezier Herman, Béatrice

(57) **Abstract**

The present invention relates to sydnone compounds substituted in position 4, to a process for the preparation of a first compound of interest C1 functionalized with a sydnone compound of the invention and to the corresponding functionalized C1 compound of interest. The present invention also relates to a process for the preparation of a conjugate of two compounds of interest C1 and C2 implying a sydnone compound according to the invention, and to the obtained conjugate. The present invention also relates to a process for preparing a compound of interest C2 comprising a strained alkyne moiety functionalized with a sydnone according to the invention and to the corresponding functionalized compound of interest C2.

## Description

### Introduction

The present invention relates to sydnone compounds substituted in position 4, to a process for the preparation of a first compound of interest C1 functionalized with a sydnone compound of the invention and to the corresponding functionalized C1 compound of interest. The present invention also relates to a process for the preparation of a conjugate of two compounds of interest C1 and C2 implying a sydnone compound according to the invention, and to the obtained conjugate. The present invention also relates to a process for preparing a compound of interest C2 comprising a strained alkyne moiety functionalized with a sydnone according to the invention and to the corresponding functionalized compound of interest C2.

### Background of the invention

The present invention relates to the domain of click chemistry (bioorthogonal reaction) and of bioconjugation of compounds of interest. Five main approaches are currently available for metal-free coupling two different compounds of interest (● and ■). These approaches are presented on the scheme below.

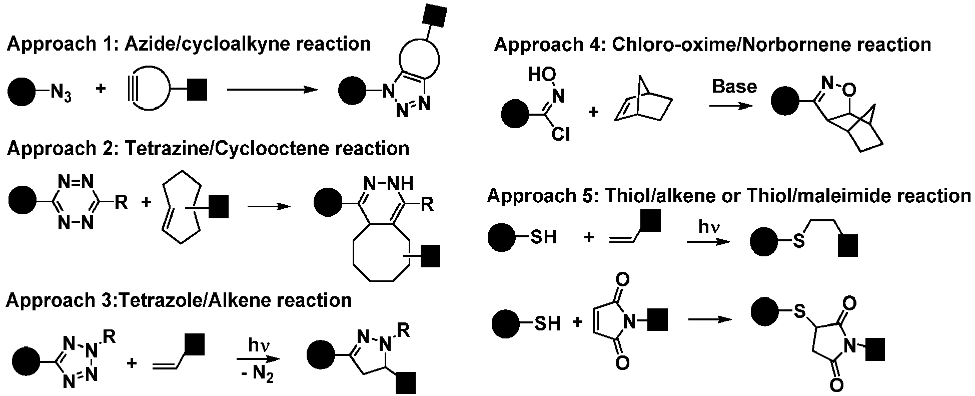

Bioconjugation reactions, for instance bioconjugation reactions involving proteins, are often performed in highly dilute conditions, for instance because of the low available amount of the starting biomaterial, or because the solubility of said starting biomaterial is low in the coupling conditions.
Consequently, it is important in the domain of biorthogonal reactions to use efficient coupling reagents allowing high yields and fast coupling kinetics.

The inventors of the present application recently discovered that reaction of a sydnone and an alkyne could be efficiently used to perform efficient bioorthogonal reactions (Kolodych et al. Angew. Chem. Int. Ed. 2013, 52, 12056-12060).
Browne et al. (J. Org. Chem. 2010, 75, 984-987) disclose the cycloaddition of 4-iodosydnones with terminal alkynes. The cycloaddition reactions disclosed by Browne et al. are performed at elevated temperatures (140°C or 200°C) in a non polar solvent (xylenes). Such conditions are not appropriate for any domain of application; for instance, such conditions are not compatible with reactions involving biomolecules, such as bioconjugation reactions. In addition, Browne et al. further disclose that this cycloaddition reaction of iodosydnones may not be transposed to bromosydnones because bromosydnones would be unstable at elevated temperatures.

Wallace et al. (Chem. Sci. 2014, published 5 February 2014) recently described the cycloaddition of phenylsydnone, which is not substituted in position 4, on a bicyclononyne.

The applicant of the present invention surprisingly evidenced that the use of a specific family of sydnone derivatives, which are substituted in position 4 with specific groups, affords a great increase in efficiency and kinetics of the coupling reactions. These sydnone compounds can be advantageously used for the conjugation of compounds of interest.

### Summary of the invention

The present invention relates to sydnone compounds of formula (I) 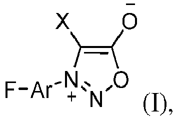 wherein
X is a halogen atom selected from the group consisting of chlorine, bromine and fluorine atoms, an aryldiazo group, an alkyl group, a phenyl group substituted with at least one electrodonating group, such as an alkoxyl group or a dialkylamino group, an alkenyl group, an alkynyl group an alkoxyl group or an amino group;
Ar is an aromatic group; and
F is a functional group allowing bonding to a compound of interest C1.

Another object of the invention is a process for preparing a functionalized compound, comprising the step of contacting a non-functionalized compound of interest C1 with a sydnone according to the invention.

Another object of the invention is a functionalized compound of interest C1 of formula (III) 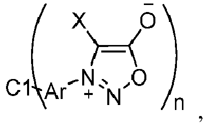 wherein the compound of formula (III) can be obtained by the process for preparing a functionalized compound according to the invention.

Another object of the invention is a process for preparing a conjugate, comprising a step of contacting a compound of interest C2 comprising a strained alkyne moiety of formula (IV) 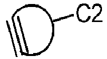 with a functionalized compound of interest C1 of formula (III) according to the invention.

Another object of the invention is a conjugate of formula (II): 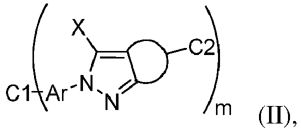 wherein C1 is a first compound of interest, and 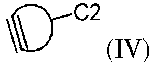 is a second compound of interest C2 comprising a strained alkyne moiety.

Another object of the invention is a process for preparing a functionalized compound, comprising the step of contacting a non-functionalized compound of interest C2 comprising a strained alkyne moiety of formula (IV) 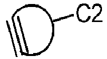 with a sydnone according to the invention.

Another object of the invention is a functionalized compound of interest C2 of formula (V) 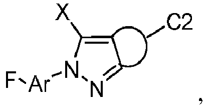 wherein said compound of formula (V) can be obtained by the process described above.

Another object of the invention is a process for preparing a conjugate, comprising a step of contacting a compound of interest C1 with a functionalized compound of interest C2 of formula (V) 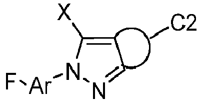 according to the invention.

### Brief description of the figures

- Figure 1:: HPLC monitoring of the kinetics of the cycloaddition reaction involving a chlorosydnone in blood plasma.
- Figure 2:: ¹H NMR spectroscopic analysis of the competitive reaction between a chlorosydnone and an azide with the cyclooctyne BCN in CD₃OD at 25°C.
- Figure 3:: Linear regressions for the kinetics of the reaction of two sydnones of the invention with BCN (bicyclononyne).
- Figure 4:: Comparison of protein labeling efficiencies with a chlorosydnone versus the corresponding non-chlorinated sydnone.

### Detailed description of the invention

A first object of the invention is a sydnone compound of formula (I): 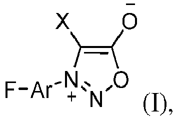 wherein
X is a halogen atom selected from the group consisting of chlorine, bromine and fluorine atoms, an aryl diazo group, an alkyl group, a phenyl group substituted with at least one electrodonating group, such as an alkoxyl group or a dialkylamino group, an alkenyl group, an alkynyl group, an alkoxyl group or an amino group;
Ar is an aromatic group; and
F is a functional group allowing bonding to a compound of interest C1.

A halogen atom is a chlorine, iodine, bromine or fluorine atom. Preferably, a halogen atom is a bromine or a chlorine atom, in particular a chlorine atom.

An alkyl group is a linear, branched or cyclic saturated hydrocarbon group comprising from 1 to 20 carbon atoms. Preferably, the alkyl group according to the invention comprises from 1 to 10, in particular from 1 to 6, carbon atoms. Examples of alkyl groups comprise methyl, ethyl, propyl, isopropyl, butyl, tertbutyl, isobutyl, n-pentyl, n-hexyl and cyclohexyl groups.
An alkenyl group (or an alkene) is a linear, branched or cyclic hydrocarbon group comprising from 2 to 20 carbon atoms and comprising at least one C=C double bond. Preferably, the alkenyl group according to the invention comprises from 2 to 10, in particular from 2 to 6, carbon atoms. Examples of alkenyl groups comprise ethylenyl, propylenyl and cyclohexenyl groups.

An alkynyl group (or an alkyne) is a linear, branched or cyclic hydrocarbon group comprising from 2 to 20 carbon atoms and comprising at least one C=C triple bond. Preferably, the alkynyl group according to the invention comprises from 2 to 10, in particular from 2 to 9, carbon atoms. Examples of alkynyl groups comprise ethynyl, propynyl, octynyl, cyclooctynyl and cyclononynyl groups.

In specific embodiments of the present invention, the alkyl, alkenyl and/or alkynyl groups may be interrupted by at least one heteroatom, preferably selected from nitrogen, oxygen and sulfur atoms.
According to the invention, the alkyl groups, alkenes and alkynes are not substituted by an aryl group.

An alkoxyl group is an alkyl group, bonded to the rest of the molecule through an oxygen atom.
An aryl diazo group is a N₂ group bonded to an aromatic group. A carboxyl group is a COOH group. A nitro group is a NO₂ group.
An amino group is a NR₁R₂ group, wherein R₁ and R₂ are independently selected from the group consisting of hydrogen atoms and alkyl groups. In an embodiment, at least one of R₁ and R₂ is an alkyl group. Such an amino group is an alkylamino group. In an embodiment, R₁ and R₂ are both alkyl groups. Such an amino group is a dialkylaminogroup.

According to the present invention, an aromatic group (or an aryl) is selected from the group consisting of:
- a phenyl group;
- a heteroaromatic group such as pyridine, thiophene, imidazole, thiazole, pyrazole, pyrole or furane; and
- a polyaromatic group such as anthracene or phenanthrene;
wherein the phenyl, heteroaromatic and/or polyaromatic group may be optionally substituted with at least one substituent (in addition to the F group), preferably selected from the group consisting of a halogen atom, an alkyl group, an alkoxyl group, a carboxyl COOH group and a nitro NO₂ group.

In an embodiment, the aromatic group is an optionally substituted phenyl group, preferably a non substituted phenyl group.

The functional group F is a functional group allowing bonding to a compound of interest C1. For instance, the functional group F may be selected from the group consisting of:
- a carboxylic acid COOH group,
- a thiol SH group,
- a maleimide 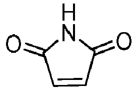 group,
- an activated ester, such as a N-hydroxysuccinimide, a N-hydrophthalimide ester, a perfluorinated ester or an acylurea,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine (-ONH₂) group,
- a hydrazine (-NH-NH₂) group,
- an azido (-N₃) group,
- a diazonium (-N₂⁺) group, optionally in presence of a counterion,
- a boronic acid -B(OR")₂ group, wherein R" is a hydrogen atom or an alkyl group,
- an isocyanate (-N=C=O) or isothiocyanate (-N=C=S) group,
- a chlorosulfonyl (-SO₂Cl) group,
- a -C≡C-C≡N group,
- an aldehyde CHO group,
- a ketone COR'" group, wherein R"' is an alkyl group as defined above, and
- an alkyl group substituted by at least one of the groups above.

The counterion can be any ion appropriate for compensating the charge of the diazonium group, and may be easily chosen by anyone of ordinary skill in the art. For instance, the couterion may be selected from the group consisting of halogenates, BF₄⁻, NO₃⁻, HSO₄⁻, PF₆⁻ CH₃COO⁻, N(SO₂CF₃)₂⁻, CF₃SO₃⁻, CH₃SO₃⁻, CF₃COO⁻, (CH₃O)(H)PO₂⁻ and N(CN)₂⁻.

When Ar is an optionally substituted phenyl group, the functional group F can be in *meta* or *para* position of the phenyl group, preferably in *para* position.
When Ar is an optionally substituted heteroaromatic or polyaromatic group, the functional group F can be in any position of the aromatic group.

In a preferred embodiment, the functional group F is selected from the group consisting of:
- a carboxylic acid COOH group,
- a thiol SH group,
- a maleimide 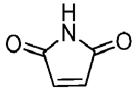 group,
- an activated ester, such as a N-hydroxysuccinimide, a N-hydrophthalimide ester, a perfluorinated ester or an acylurea,
- an amino (-NRR') group, wherein R and R' are independently hydrogen atoms, alkyl, alkene, alkyne or aryl groups as defined above,
- a hydroxylamine (-ONH₂) group,
- a hydrazine (-NH-NH₂) group,
- an azido (-N₃) group,
- an isocyanate (-N=C=O) or isothiocyanate (-N=C=S) group,
- a chlorosulfonyl (-SO₂Cl) group,
- a -C≡C-C≡N group, and
- an alkyl group substituted by at least one of the groups above.

In a highly preferred embodiment, the functional group F is selected from the group consisting of a carboxylic acid COOH group, an activated ester, such as a N-hydroxysuccinimide, a N-hydrophthalimide ester, a perfluorinated ester or an acylurea, and an alkyl group substituted by at least one of these groups.

A compound of interest may be for instance a molecule, such as a fluorophore, for instance rhodamine, a group of atoms comprising at least one radioactive atom (¹⁴C, ³H, or ¹³¹I for instance), a group of atoms of known mass (a mass tag), a ligand, a drug, a therapeutic agent, a biomolecule, such as an antibody, a protein, such as BSA (bovine serum albumin), a DNA fragment, a nanoobject, such as a nanoparticle (*ie* an object or a particle of 0.1 to 1000 nm), or a support, such as a polymer.

In an embodiment, X is selected from the group consisting of a halogen atom selected from the group consisting of chlorine, bromine and fluorine atoms, an aryl diazo group, an alkyl group, a phenyl group substituted with at least one electrodonating group, such as an alkoxyl group or a dialkylamino group, an alkenyl group, an alkynyl group and an amino group.

In a preferred embodiment, X is a halogen atom selected from the group consisting of chlorine, bromine and fluorine atoms, in particular a chlorine or bromine atom.
In another preferred embodiment, X is an alkenyl group, an alkynyl group, or an aryldiazo group.

The sydnones of the invention may be synthesized by any appropriate method known by one of ordinary skill in the art.
For instance, the scheme below presents different synthesis routes for sydnones according to the invention. The Ar groups substituting the nitrogen atom of the sydnones may be substituted with a F group.

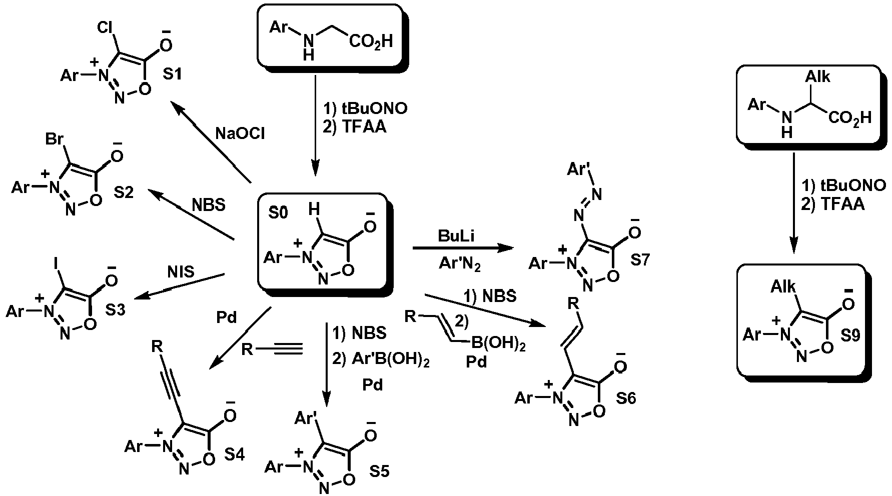

Another object of the invention is a process for the preparation of a functionalized compound C1, comprising the step of contacting a non-functionalized compound of interest C1 with a sydnone according to the invention.

In the present invention, the term "functionalized compound" refers to a compound that is bonded to a sydnone compound according to the invention.

The compound of interest C1 is preferably a compound as defined above with at least one group able to covalently bond to a F group, such as
- a carboxylic acid COOH group,
- a thiol SH group,
- a maleimide 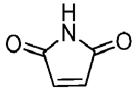 group,
- an activated ester, such as a N-hydroxysuccinimide, a N-hydrophthalimide ester, a perfluorinated ester or an acylurea,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine (-ONH₂) group,
- a hydrazine (-NH-NH₂) group,
- an azido (-N₃) group,
- a diazonium (-N₂⁺) group, optionally in presence of a counterion,
- a boronic acid -B(OR")₂ group, wherein R" is a hydrogen atom or an alkyl group,
- an isocyanate (-N=C=O) or isothiocyanate (-N=C=S) group,
- a chlorosulfonyl (-SO₂Cl) group,
- a -C≡C-C≡N group,
- an aldehyde CHO group,
- a ketone COR'" group, wherein R"' is an alkyl group as defined above, or
- an alkyl group substituted by at least one of the groups above.

In an embodiment, the group able to covalently bond to a F group of C1 is selected from the group consisting of:
- a carboxylic acid COOH group,
- a thiol SH group,
- a maleimide 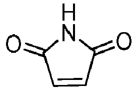 group,
- an activated ester, such as a N-hydroxysuccinimide, a N-hydrophthalimide ester, a perfluorinated ester or an acylurea,
- an amino (-NRR') group, wherein R and R' are independently hydrogen atoms, alkyl, alkene, alkyne or aryl groups as defined above,
- a hydroxylamine (-ONH₂) group,
- a hydrazine (-NH-NH₂) group,
- an azido (-N₃) group,
- an isocyanate (-N=C=O) or isothiocyanate (-N=C=S) group,
- a chlorosulfonyl (-SO₂Cl) group,
- a -C≡C-C≡N group, and
- an alkyl group substituted by at least one of the groups above.

In a preferred embodiment, the group able to covalently bond to a F group of C1 is selected from the group consisting of a carboxylic acid COOH group, an activated ester, such as a N-hydroxysuccinimide, a N-hydrophthalimide ester, a perfluorinated ester or an acylurea, and an alkyl group substituted by at least one of these groups.

If the compound of interest C1 comprises several groups able to covalently bond to F groups, the process may prepare a compound of interest C1 functionalized with several sydnones.

The process of preparation of a functionalized compound of interest C1 according to the invention may comprise a preliminary step of covalent bonding of at least one moiety comprising a reactive group to C1, wherein the reactive group is able to covalently bond to the functional group F of the sydnone compound according to the invention.

Another object of the invention is a functionalized compound of interest C1 of formula 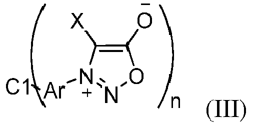 obtainable, preferably obtained, by the process for the preparation of a functionalized compound of interest C1 according to the invention, wherein n is an integer and preferably ranges from 1 to 100. In an embodiment, n is from 1 to 50, preferably from 1 to 30. In a specific embodiment, n is 1.

Another object of the invention is a process for the preparation of a conjugate, preferably a conjugate of formula (II) 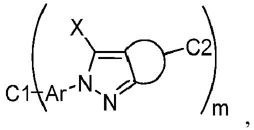 comprising a step of contacting a compound of interest C2 comprising a strained alkyne moiety of formula (IV) 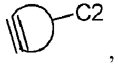 preferably a strained cyclic alkyne moiety, in particular a cyclooctyne moiety, with a functionalized compound of interest C1 according to the invention.

The process for the preparation of a conjugate may comprise a preliminary step of covalent bonding of a moiety comprising a strained alkyne to the compound of interest C2.

According to the present invention, the terms "strained alkyne" refer to an alkyne wherein the triple bond is sterically strained, such as a mono or multi cyclic alkyne comprising from 6 to 12 carbon atoms. For instance, the strained alkyne may be a cyclooctyne, in particular it is bicyclononyne BCN.

The sydnone compounds of the invention can be used advantageously in order to couple both compounds of interest C1 and C2.
In an embodiment, C1 is a fluorophore or a group of atoms comprising at least one radioactive atom, and C2 is a biomolecule, a nanoobject or a polymer comprising, or functionalized with, a strained alkyne moiety. In this embodiment, the sydnone of the invention affords the labelling of the biomolecule, nanoobject or polymer. In a particular embodiment, C2 is an antibody and this embodiment leads to the formation of a labelled antibody.
In another embodiment, C1 is a nanoobject or a biomolecule, and C2 is a biomolecule, such as an antibody. In this embodiment, the sydnone of the invention affords the bioconjugation of the first nanoobject or biomolecule and the second biomolecule.
In another embodiment, C1 is a therapeutic agent, and C2 is a biomolecule, such as an antibody. In this embodiment, the sydnone of the invention affords the bioconjugation therapeutic agent and the biomolecule. In a particular embodiment, C2 is an antibody and this embodiment leads to the formation of a therapeutic antibody.
One of the main goals of the present invention is to couple two different compounds of interest C1 and C2. Consequently, the nature of C1 and C2 can be inverted. This means that the invention also encompasses the (C2, C1) couples of compounds of interest even if the examples provided above are for (C1, C2) couples.

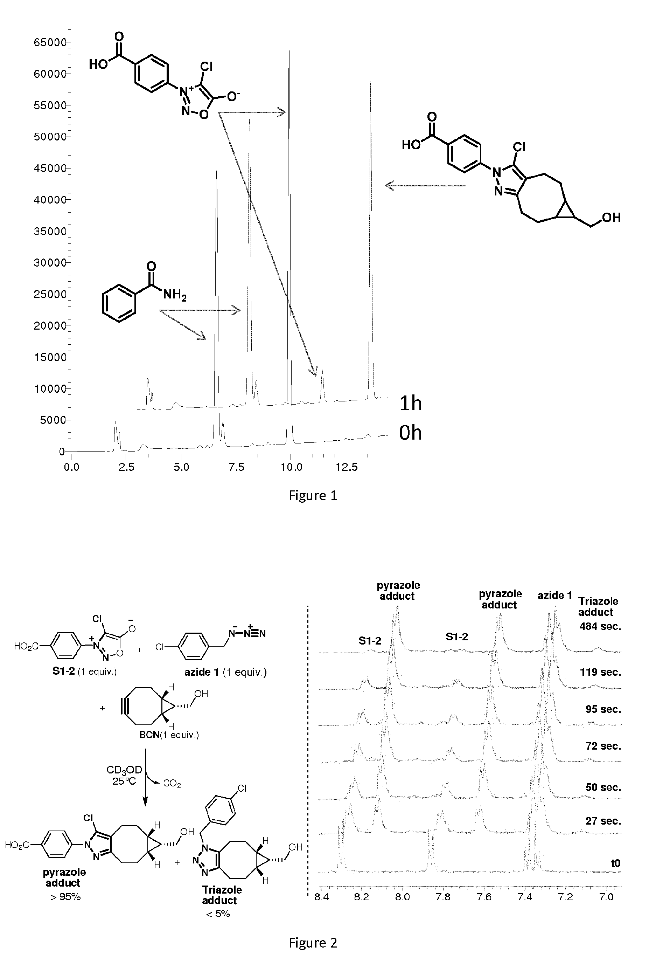

The sydnone compounds of the invention and the compounds of interest C1 functionalized with a sydnone according to the invention can be coupled very efficiently to strained alkynes, such as cyclic alkynes, in particular cyclooctynes. The corresponding coupling products are obtained with higher yields and faster kinetics than with the current competitive coupling systems. For instance, the applicant has unexpectedly proven that these compounds react up to 30 times faster than the non substituted sydnone disclosed by Wallace on strained cyclic alkynes.
The coupling reaction implies the formation of a cycloadduct formed by a [3+2] cyclization, said cycloadduct then turning to a stable pyrazole by retro-Diels Alder reaction, triggering release of CO₂. The reaction of a sydnone compound according to the invention with a cycloalkyne is represented as example on the scheme below. 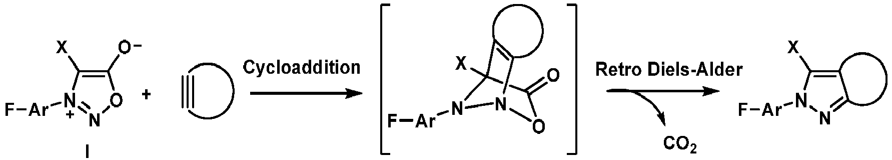 The present compounds allow the efficiency of the reaction to be maintained in biological media, such as culture media, cell lysates or plasma. In addition, as the coupling reaction involving the sydnones of the invention is very rapid, in particular when compared to the other possible coupling reactions, for instance the other coupling reactions used in click-chemistry, the reaction can be efficiently performed even in highly dilute conditions, which is classically the case for instance for bioconjugation reactions. For example, the sydnones of the invention allow high level of fluorescent labeling of a protein used at 50 µg/mL in only 5 min, when only a lower amount of labeling could be obtained even after 16 hours with a non substituted sydnone as disclosed by Wallace (example 4). Taking into account this result, the sydnones of the invention would allow coupling reactions on proteins at concentration below 1 µg/mL. The superior activity of sydnones according to the invention, such as chlorosydnones, is also illustrated in example 3 and figure 2 which correspond to a competitive coupling reaction towards a cyclooctyne with an azide compound (which reacts faster than non substituted sydnones). Competition is in favor of the chlorosydnone for more than 95%.

Another object of the invention is a conjugate of formula (II): 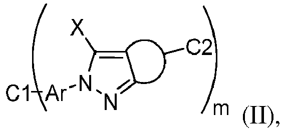 wherein C1 is a first compound of interest, 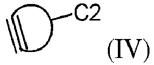 is a second compound of interest C2 comprising a strained alkyne moiety and m is an integer and preferably ranges from 1 to 100. m is inferior or equal to n as defined above. In an embodiment, m is equal to n. In a specific embodiment, m is 1.

Another object of the invention is a process for the preparation of a functionalized compound C2 comprising a strained alkyne moiety, comprising the step of contacting a non-functionalized compound of interest C2 with a sydnone according to the invention. The compound of interest C2 is as disclosed above.
The process for the preparation of a functionalized compound C2 may comprise a preliminary step of covalent bonding of a strained alkyne moiety to the compound of interest C2.

Another object of the invention is a functionalized compound of interest C2 of formula 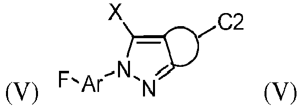 obtainable, preferably obtained, by the process of the functionalization according to the invention.

Another object of the invention is a process for the preparation of a conjugate, preferably a conjugate of formula (II) 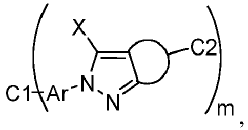 comprising a step of contacting a compound of interest C1 with a functionalized compound of interest C2 of formula (V) according to the invention.

The conjugate of formula (II) 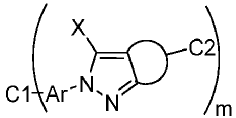 is preferably obtained by one of the processes according to the invention.

The following examples are provided as illustrative, and not limitative, of the present invention.

### Examples

### Example 1: Synthesis of sydnones according to the invention

In the following examples, the provided yields are molar yields unless specified differently.

### Synthesis of sydnone S2-2: 4-bromo-3-(4-carboxyphenyl)-1,2,3λ⁵-oxadiazol-3-ylium-5-olate

### a) Synthesis of sydnone S0-4 (3-(4-carboxyphenyl)-1,2,3λ⁵-oxadiazol-3-ylium-5-olate)

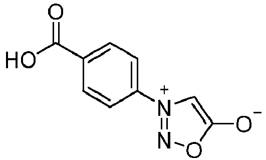

To a vigorously stirred suspension of *p*CO₂H-phenyl glycine (1.58 g, 8.10 mmol) in 10% aqueous HCl (8.10 mL) at 0°C was added dropwise, over a period of 40 min, a solution of NaNO₂ (0.56 g, 8.10 mmol) in water (8.10 mL). The resulting mixture was stirred at room temperature under argon for 14 h. The precipitate was collected by filtration, washed with small amount of methanol and dried to obtain 1.54 g (6.90 mmol, 85%) of the intermediate nitroso compound. The later (1.30 g, 5.80 mmol) was stirred at 100°C for 3 h in acetic anhydride (6.70 mL). The resulting solution was concentrated under *vacuum.* The residue was triturated with water (10 mL), the precipitate was collected by filtration and recrystallized from methanol. 622 mg (3.00 mmol, 52%) of compound **S0-4** were isolated as a white solid.

¹H NMR (400 MHz, DMF-d₇, δ ppm): 8.32 (d, *J* = 8.5 Hz, 2H), 8.19 (d, *J* = 8.5 Hz, 2H), 7.88 (s, 1H). ¹³C NMR (100 MHz, DMF-d₇, δ ppm): 169.7, 167.1, 138.9, 135.7, 132.3, 123.1, 96.1. LCMS (ESI) *m*/*z:* 207 [M+H]⁺. Mp.: 232-234 °C.

### b) Synthesis of sydnone S2-2

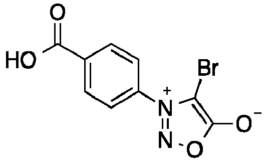

N-Bromosuccinimide (1.1 eq) was added to a stirred solution of sydnone S0-4 (0.50 mmol, 1 eq) in 1.2 mL of acetic acid. The mixture was allowed to stir at room temperature for 2h after which time 5 mL of water was added. The resultant precipitate was isolated by filtration to afford the brominated compound as an orange solid (44%).

¹H NMR (400 MHz, Acetone-d₆, δ ppm): 8.39 (d, J = 8.6 Hz, 2H), 8.03 (d, J = 8.6 Hz, 2H). ¹³C NMR (100 MHz, Acetone-d₆, δ ppm): 166.1, 166.0, 138.3, 135.3, 132.1, 126.7, 85.9. IR (KBr, cm⁻¹): 2820, 2087, 1942, 1904, 1850, 1743, 1606, 1591, 1509, 1445, 1423, 1335, 1317, 1290, 1205, 1127, 1114, 1035, 1018, 974, 942, 881, 858, 820, 808. LCMS (ESI): *m*/*z:* 285 [M(⁷⁹Br)+H]⁺, 287 [M(⁸¹Br)+H]⁺. Mp.: 117-119 °C (decomp.)

### Synthesis of Sydnone S1-2: 3-(4-carboxyphenyl)-4-chloro-1,2,3λ⁵-oxadiazol-3-ylium-5-olate

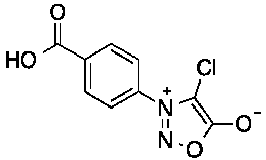

A solution of sodium hypochlorite 10% (1.23 mL, 2 mmol) was added dropwise to a stirred solution of carboxyphenylsydnone (206 mg, 1.00 mmol) in a dioxane/HCl 1M 2/1 mixture (12 mL).The mixture was allowed to stir at room temperature for 4 h. The mixture was poured in a solution of Na₂S₂O₃ 20%. EtOAc was added and the aqueous layer was extracted 3 times with EtOAc. The organic layers were combined, dried over MgSO₄ and concentrated under reduced pressure. The crude product was purified by silica gel column to afford the chlorinated compound as an orange solid (40%).

¹H NMR (400 MHz, Acetone-d₆, δ ppm): 8.30 (d, J = 8.6 Hz, 2H), 7.90 (d, J=8.6 Hz, 2H). IR (KBr, cm⁻¹): 3315, 2927, 2549, 1791, 747, 1691, 1607, 1510, 1455, 1425, 1346, 1320, 1290, 1219, 1176, 1128, 1113, 1043, 1018, 182, 972, 945, 886, 859, 808. LCMS (ESI): *m*/*z:* 241 [M(³⁵Cl)+H]⁺, 243 [M(³⁷Cl)+H]⁺.

### Synthesis of 4-bromo-3-(4-nitrophenyl)-1,2,3λ⁵-oxadiazol-3-ylium-5-olate

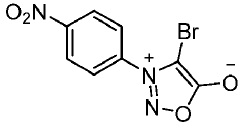

N-Bromosuccinimide (97.9 mg, 0.55 mmol) was added to a stirred solution of nitrophenylsydnone (104 mg, 0.50 mmol) in 1.2 mL of acetic acid. The mixture was allowed to stir at room temperature for 2h after which time 5 mL of water was added. The resultant precipitate was isolated by filtration to afford 4-bromo-3-(4-nitrophenyl)-1,2,3λ⁵-oxadiazol-3-ylium-5-olate (70%).

¹H NMR (400 MHz, Acetone-d₆, δ ppm): 8.39 (d, J = 8.6 Hz, 2H), 8.03 (d, J = 8.6 Hz, 2H). ¹³C NMR (100 MHz, Acetone-d₆, δ ppm): 166.1, 166.0, 138.3, 135.3, 132.1, 126.7, 85.9. IR (KBr, cm⁻¹): 3112, 3091, 3066, 1945, 1937, 1907, 1855, 1755, 1614, 1594, 1527, 1496, 1449, 1374, 1344, 1315, 1294, 1204, 1164, 1105, 1030, 1013, 195, 966, 885, 861, 848. LCMS (ESI): *m*/*z:* 286 [M(⁷⁹Br)+H]⁺, 288 [M(⁸¹Br)+H]⁺. Mp.: 150-152 °C.

### Synthesis of 3-(4-nitrophenyl)-1,2,3λ⁵-oxadiazol-3-ylium-5-olate

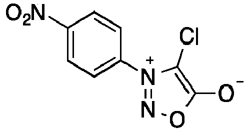

N-Chlorosuccinimide (134 mg, 1.00 mmol) was added to a stirred solution of nitrophenylsydnone (104 mg, 0.50 mmol) in 1.2 mL of acetic acid. The mixture was allowed to stir at room temperature for 6 h. The crude product was purified by silica gel column chromatography (eluent: Dichloromethane/ MeOH 99/1, AcOH 1%) to afford 3-(4-nitrophenyl)-1,2,3λ⁵-oxadiazol-3-ylium-5-olate.

¹H NMR (400 MHz, CDCl₃, δ ppm): 8.29 (m, 2H), 8.14 (m, 2H). IR (KBr, cm⁻¹): 3315, 3123, 3054, 2639, 2500, 1935, 1773, 1721, 1659, 1610, 1595, 1551, 1513, 1493, 1418, 1409, 1389, 1376, 1341, 1328, 1302, 1275, 1244, 1203, 1175, 1111, 981, 855, 814.

### Example 2: Kinetic study of the reaction of sydnones according to the invention with cyclic alkynes

Reactions of sydnone S1-2 with bicyclononyne BCN was carried out in blood plasma at 100 µM concentration of sydnone and 150 µM concentration of cyclooctyne BCN using the following procedure:
To 900 µL of blood plasma were added 10 µL of the solution of benzamide (internal standard, 100 mM in DMSO), 1 µL of the solution of sydnone (100 mM in DMSO) and 1.5 µL of the solution of BCN (100mM in DMSO). The reaction mixture was injected in HPLC every 30 min and the conversion was followed by measuring the normalized sydnone peak area.

Figure 1 presents the HPLC monitoring of the reaction between sydnone S1-2 of the invention and the cyclic alkyne BCN. The reaction has been conducted at 100 µM in blood plasma. The results indicated an almost complete reaction after 1 hour in blood plasma, proving that the reaction is fast enough to be useful to bioconjugation application in complex biological media.

### Example 3: Comparison with the coupling with azides

0.1 mmol of sydnone S1-2, azide 1 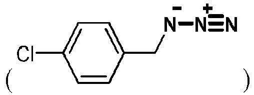 and BCN were dissolved in CD₃OD at room temperature. The reaction was followed by NMR on the aromatic part of the spectrum which provides specific NMR signals.

Figure 2 presents the evolution of the NMR spectrum in function of time of a competitive cycloaddition reaction between the sydnone of the invention S1-2 and an azide with the cyclooctyne BCN. The results clearly confirmed the superior activity of the chlorosydnone over azide to undergo reaction with BCN.

### Example 4: Kinetic study of the reaction of different sydnones with cyclic alkynes

The kinetics of the coupling reaction of different sydnones with two cyclic alkynes was studied, according to the following scheme.

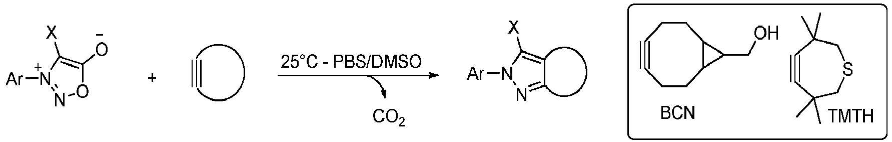

Reactions of sydnones with BCN (or TMTH) were carried out in PBS/DMSO (9:1) mixtures at 100 µM concentration of sydnones and 150 µM concentration of BCN using the following procedure:
To 900 µL of phosphate buffered saline (PBS, 100mM) was added 87.5 µL of DMSO, 10 µL of the solution of benzamide (internal standard, 100 mM in DMSO), 1 µL of the solution of sydnone (100mM in DMSO) and 1.5 µL of the solution of BCN (100mM in DMSO). The reaction mixture was injected in HPLC every 30 min and the conversion was followed by measuring the normalized sydnone peak area.

Table 1 below presents the kinetic constant values K for these reactions, depending on the Ar group and the X group. Sydnones S1-2 and S2-2 are according to the invention.

**Table 1. K unit is M⁻¹.sec⁻¹, n.d. = not determined**

| Sydnone | Ar | X | K (BCN) | K (TMTH) |
|---|---|---|---|---|
| **S0-1** | *p*OMeC₆H₄ | H | 0.006 ± 0.001 | 28.4 |
| **S0**-**2** | *p*MeC₆H₄ | H | 0.032 ± 0.001 | n.d. |
| **S0-3** | C₆H₅ | H | 0.027 ± 0.002 | 183 |
| **S0-4** | *p*CO₂HC₆H₄ | H | 0.059 ± 0.001 | n.d. |
| **S0-5** | *p*CF₃C₆H₄ | H | 0.199 ± 0.002 | n.d. |
| **S0-6** | *p*NO₂C₆H₄ | H | 0.289 ± 0.012 | n.d. |
| **S9-1** | C₆H₅ | CH₃ | 0.018 ± 0.002 | 16.1 |
| **S5-1** | C₆H₅ | C₆H₅ | 0.027 ± 0.001 | n.d. |
| **S5-2** | C₆H₅ | *p*OMeC₆H₄ | 0.015 ± 0.001 | n.d. |
| **S6-1** | C₆H₅ | 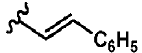 | 0.015 ± 0.001 | n.d. |
| **S4-1** | C₆H₅ | 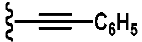 | 0.113 ± 0.001 | n.d. |
| **S8-1** | C₆H₅ | SMe | 0.044 ± 0.001 | n.d. |
| **S7-1** | C₆H₅ | 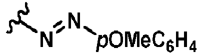 | 0.02 ± 0.001 | n.d. |
| **S1-1** | C₆H₅ | Cl | 0.872 ± 0.034 | n.d. |
| **S2-1** | C₆H₅ | Br | 0.592 ± 0.021 | n.d. |
| **S3-1** | C₆H₅ | I | 0.306 ± 0.008 | n.d. |
| **S2-2** | *p*CO₂HC₆H₄ | Br | 0.798 ± 0.065 | 101 |
| **S1-2** | *p*CO₂HC₆H₄ | Cl | 1.593 ± 0.034 | 322 |

These results clearly show that the sydnones comprising a halogen atom in position 4 afford a faster reaction than the sydnones comprising no substitution or another substituent in position 4. In particular, 4-chloro-sydnones were found to react with the cyclooctyne BCN more than approximately 30 times faster than sydnones that are non substituted in position 4. Actually, the ratios of the K constants of sydnones according to the invention to the K constants of the corresponding sydnones that are not substituted in position 4 are the following: K(S1-1)/K(S0-3) = 32, and K(S1-2)/K(S0-4)=27.

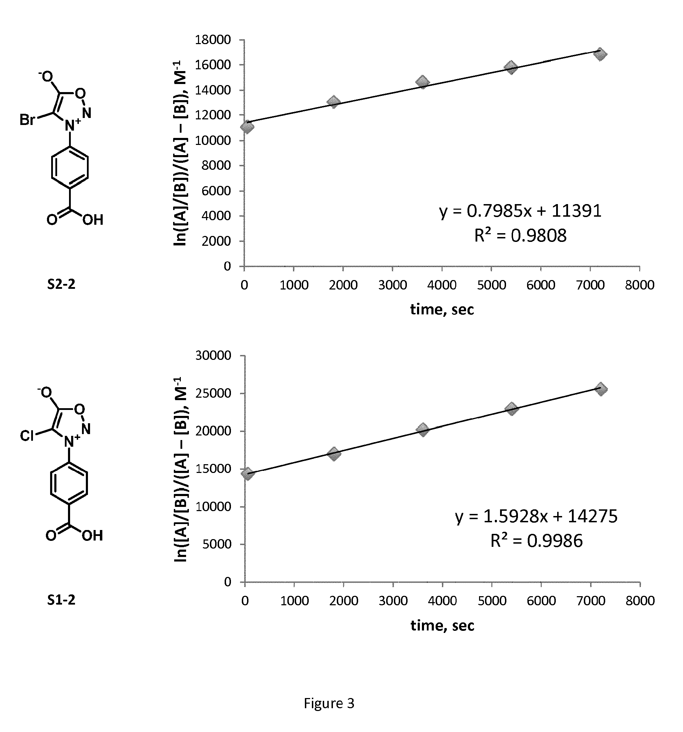

Second order reaction rate was determined by plotting ln([A]/[B])/([A] - [B]) versus time and analyzing by linear regression. Second order rate constant corresponds to the determined slope. Linear regression curves for the sydnones of the invention are presented in figure 3.

### Example 4: Coupling of a sydnone according to the invention with a protein as compound of interest C1 and its use for fluorescence labelling with a rhodamine derivative as compound of interest C2.

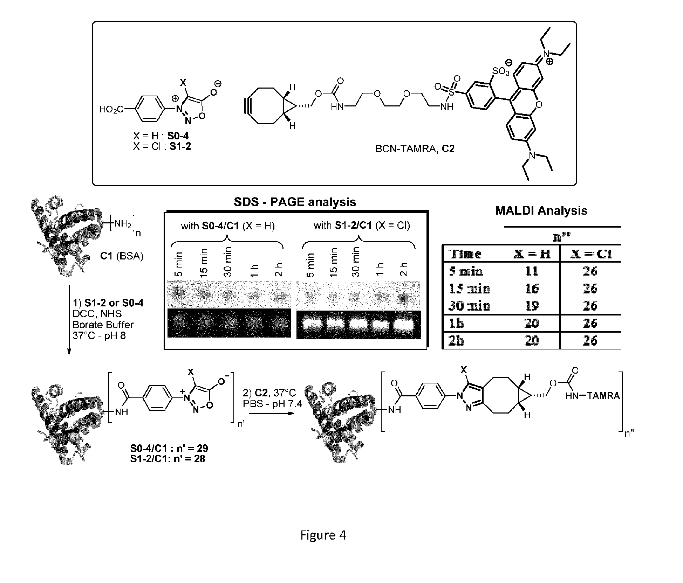

The sydnone **S1-2** of the invention was coupled to bovin serum albumin (BSA), used as model protein, by activation of the F function of the sydnone followed by standard peptide coupling to the protein. The corresponding protein-sydnone conjugate was then reacted with a rhodamine (TAMRA)-cyclooctyne conjugate. Sydnone **S0-4** which has no substituent in position 4 was also used for comparison. The SDS-PAGE and MALDI results presented in figure 4 clearly indicate a better efficiency and a better kinetic of the sydnone of the invention for the rhodamine labeling of the protein.

### Preparation of BSA-Sydnone conjugates S1-2/C1 and S0-4/C1:

To a solution of N-hydroxysuccinimide (25 µL, 44.64 µmol, 1 eq) in DMF was added a solution of dicyclohexylcarbodiimide (25 µL, 44.64 µmol, 1 eq) in DMF and a solution of sydnone (250 µL, 44.64 µmol) in DMF. The mixture was stand at room temperature during 1h and was directly used for the conjugation with BSA protein.

A solution of BSA (250 µL, 72 nmol) in phosphate buffer (100 mM, pH 7.4) and 950 µL of borax buffer (0.05 M, pH 9.2) were added to the previous solution. The resulting mixture was incubated during 1 h at 37 °C and purified by size-exclusion chromatography on Sephadex® G-25 medium gel (eluted with 5 mM potassium phosphate buffer pH 8). 29 **S0-4** residues and 28 **S1**-**2** residues per BSA protein were conjugated according to MALDI TOF-MS analyses.

### Preparation of BSA-Sydnone-cycloalkyne-TAMRA conjugates:

A solution of BCN-POE3-NH-lissamine rhodamine B conjugate (51.2 µL, 130 nmol, 1 eq/sydnones) in MeOH was added to a solution of the BSA-sydnone conjugate (50 µg/mL) in phosphate buffer (5 mM, pH 7.4) and stand at room temperature. Sampling for electrophoresis and MALDI-TOF analysis were performed at 5 min, 15 min, 30 min, 1 h and 16 h. The samples were concentrated with a centrifugal filter unit Amicon® Ultra Millipore™ Ultracel® -10K and frozen to -80°C.

MALDI-TOF-MS analyses were performed on Voyager™ Biospectrometry™ Workstation. SDS-PAGE was performed on PhastGel™ GE Healthcare™ Gradient 10-15 gel using PhastGel™ GE Healthcare™ SDS buffer Strips. Fluorescence was visualized on Molecular Imager® VersaDoc™ MP 4000 system prior to staining with Coomassie Blue.

## Claims

1. Sydnone compound of formula (I) 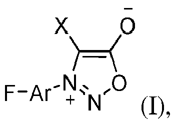 wherein
- X is a halogen atom selected from the group consisting of chlorine, bromine and fluorine atoms, an aryldiazo group, an alkyl group, a phenyl group substituted with at least one electrodonating group, an alkenyl group, an alkynyl group, an alkoxyl group or an amino group,
- Ar is an aromatic group, and
- F is a functional group allowing bonding to a compound of interest C1.

2. Sydnone compound according to claim 1, wherein X is a halogen atom selected from the group consisting of chlorine, bromine and fluorine atoms.

3. Sydnone compound according to claim 1, wherein X is an alkenyl group, an alkynyl group or an aryldiazo group.

4. Sydnone compound according to anyone of claims 1 to 3, wherein the aromatic group is selected from the group consisting of:
- a phenyl group;
- a heteroaromatic group; and
- a polyaromatic group;
wherein the phenyl, heteroaromatic and/or polyaromatic group may be optionally substituted with at least one substituent selected from the group consisting of a halogen atom, an alkyl group, an alkoxyl group, a carboxyl COOH group and a nitro NO₂ group.

5. Sydnone compound according to anyone of claims 1 to 4, wherein the functional group F is selected from the group consisting of:
- a carboxylic acid COOH group,
- a thiol SH group,
- a maleimide 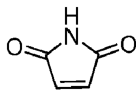 group,
- an activated ester, such as a N-hydroxysuccinimide, a N-hydrophthalimide ester, a perfluorinated ester or an acylurea,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine (-ONH₂) group,
- a hydrazine (-NH-NH₂) group,
- an azido (-N₃) group,
- a diazonium (-N₂⁺) group, optionally in presence of a counterion,
- a boronic acid -B(OR")₂ group, wherein R" is a hydrogen atom or an alkyl group,
- an isocyanate (-N=C=O) or isothiocyanate (-N=C=S) group,
- a chlorosulfonyl (-SO₂Cl) group,
- a -C≡C-C≡N group,
- an aldehyde CHO group,
- a ketone COR"' group, wherein R'" is an alkyl group as defined above, and
- an alkyl group substituted by at least one of the groups above.

6. Sydnone compound according to anyone of claims 3 to 5, wherein X is a phenyl group substituted with at least one electrodonating group selected from an alkoxyl group and a dialkyamino group.

7. Process for the preparation of a functionalized compound, comprising the step of contacting a non-functionalized compound of interest C1 with a sydnone compound according to anyone of claims 1 to 6.

8. Functionalized compound of interest C1 of formula (III) 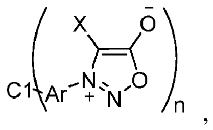 wherein n is an integer from 1 to 100, obtainable by the process of functionalization according to claim 7.

9. Process for the preparation of a conjugate, comprising a step of contacting a compound of interest C2 comprising a strained alkyne moiety with the functionalized compound of interest C1 according to claim 8.

10. Conjugate of formula (II): 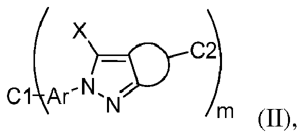 wherein C1 is a first compound of interest, 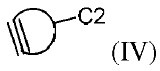 is a second compound of interest C2 comprising a strained alkyne moiety, and m is an integer from 1 to 100.

11. Conjugate according to claim 10, wherein C1 is a fluorophore and C2 is a biomolecule, or C1 is a therapeutic agent and C2 is a biomolecule.

12. Conjugate according to claim 11, wherein the biomolecule is an antibody.

13. Process for the preparation of a functionalized compound C2 comprising a strained alkyne moiety, comprising the step of contacting a non-functionalized compound of interest C2 with a sydnone according to anyone of claims 1 to 6.

14. Functionalized compound of interest C2 of formula (V) 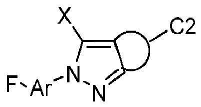 obtainable by the process of preparation according to claim 13.

15. Process for the preparation of a conjugate, comprising a step of contacting a compound of interest C1 with a functionalized compound of interest C2 of formula (V) according to claim 14.
